(19) **Europäisches Patentamt / European Patent Office / Office européen des brevets**

(11) **EP 1 649 287 B1**

(12) **EUROPEAN PATENT SPECIFICATION**

(45) Date of publication and mention
of the grant of the patent:
**06.08.2008 Bulletin 2008/32**

(51) Int Cl.:
*G01N 33/551* (2006.01)    *G01N 33/50* (2006.01)
*G01N 33/68* (2006.01)    *A61K 39/00* (2006.01)

(21) Application number: **04739011.7**

(22) Date of filing: **28.07.2004**

(86) International application number:
**PCT/DK2004/000514**

(87) International publication number:
**WO 2005/022157 (10.03.2005 Gazette 2005/10)**

(54) **EVALUATION OF ADJUVANTED VACCINES**

EVALUIERUNG ADJUVANSHALTIGER IMPFSTOFFE

EVALUATION DE VACCINS CONTENANT DES ADJUVANTS

(84) Designated Contracting States:
**AT BE BG CH CY CZ DE DK EE ES FI FR GB GR HU IE IT LI LU MC NL PL PT RO SE SI SK TR**
Designated Extension States:
**LT LV**

(30) Priority: **05.08.2003 US 493020 P**
**05.08.2003 DK 200301130**

(43) Date of publication of application:
**26.04.2006 Bulletin 2006/17**

(73) Proprietor: **ALK-Abelló A/S**
**2970 Hørsholm (DK)**

(72) Inventors:
• **WÜRTZEN, Peter, Adler**
**DK-2840 Holte (DK)**
• **LUND, Gitte**
**DK-3450 Allerød (DK)**
• **JACOBI, Henrik Hugo**
**DK-2950 Vedbaek (DK)**
• **IPSEN, Hans-Henrik**
**DK-3400 Hillerød (DK)**

(74) Representative: **Olsen, Lars Pallisgaard**
**Zacco Denmark A/S**
**Hans Bekkevolds Allé 7**
**2900 Hellerup (DK)**

(56) References cited:
**US-A- 4 127 385**

• **KATZ J B ET AL: "IN VITRO ASSESSMENT OF VIRAL ANTIGEN CONTENT IN INACTIVATED ALUMINUM HYDROXIDE ADJUVANTED VACCINES" JOURNAL OF VIROLOGICAL METHODS, AMSTERDAM, NL, vol. 25, no. 1, 1989, pages 101-108, XP001009902 ISSN: 0166-0934**
• **THRAENHART O ET AL: "STANDARDIZATION OF AN ENZYME IMMUNOASSAY FOR THE IN-VITRO POTENCY ASSAY OF INACTIVATED TISSUE CULTURE RABIES VACCINES DETERMINATION OF THE RABIES VIRUS GLYCOPROTEIN WITH POLYCLONAL ANTISERA" JOURNAL OF BIOLOGICAL STANDARDIZATION, vol. 17, no. 4, 1989, pages 291-310, XP008029516 ISSN: 0092-1157**
• **DATABASE WPI Section Ch, Week 199326 Derwent Publications Ltd., London, GB; Class B04, AN 1993-212602 XP002170319 & SU 1 746 318 A (POLIOMYELITIS VIRUS INST) 7 July 1992 (1992-07-07) cited in the application**
• **KATZ J: "Desorption of porcine parvovirus from aluminum hydroxide adjuvant with subsequent viral immunoassay or hemagglutination assay." VETERINARY RESEARCH COMMUNICATIONS. NETHERLANDS 1987, vol. 11, no. 1, 1987, pages 83-92, XP008029515 ISSN: 0165-7380**
• **LACEY C J N ET AL: "Phase IIa safety and immunogenicity of a therapeutic vaccine, TA-GW, in persons with genital warts" JOURNAL OF INFECTIOUS DISEASES, vol. 179, no. 3, March 1999 (1999-03), pages 612-618, XP002277071 ISSN: 0022-1899**

EP 1 649 287 B1

- KRISHNAN LAKSHMI ET AL: "Archaeosome vaccine adjuvants induce strong humoral, cell-mediated, and memory responses: Comparison to conventional liposomes and alum" INFECTION AND IMMUNITY, AMERICAN SOCIETY FOR MICROBIOLOGY. WASHINGTON, US, vol. 68, no. 1, January 2000 (2000-01), pages 54-63, XP002166447 ISSN: 0019-9567
- DESBORDES J: "Problems raised by potency evaluation of allergen preparations." DEVELOPMENTS IN BIOLOGICAL STANDARDIZATION. SWITZERLAND 1975, vol. 29, 1975, pages 219-226, XP008029606 ISSN: 0301-5149
- HOFFMANN A ET AL: "Determination of the allergenic activity of birch pollen and apple prick test solutions by measurement of beta-hexosaminidase release from RBL-2H3 cells. Comparison with classical methods in allergen standardization." ALLERGY. DENMARK MAY 1999, vol. 54, no. 5, May 1999 (1999-05), pages 446-454, XP002277072 ISSN: 0105-4538
- HOGENESCH H: "Mechanisms of stimulation of the immune response by aluminum adjuvants" VACCINE, BUTTERWORTH SCIENTIFIC. GUILDFORD, GB, vol. 20, 31 May 2002 (2002-05-31), pages S34-S39, XP004361371 ISSN: 0264-410X
- DEMOLY P ET AL: "Allergen-induced mediator release tests." ALLERGY. DENMARK JUL 2003, vol. 58, no. 7, July 2003 (2003-07), pages 553-558, XP002277073 ISSN: 0105-4538
- LEVINGS RANDALL L ET AL: "In vitro potency assays for nonreplicating veterinary vaccines: Comparison to in vivo assays and considerations in assay development" VETERINARY MICROBIOLOGY, vol. 37, no. 3-4, 1993, pages 201-219, XP002277074 Conference on Characterization and Quantitation of Immunogens in Veterinary Biologics;Ames, Iowa, USA; May 5-6, 1992 ISSN: 0378-1135
- GEHLHAR K ET AL: "Characterization of Modified Allergen Extracts by in vitro beta-Hexosaminidase Release from Rat Basophils" INT ARCH ALLERGY IMMUNOL, vol. 136, pages 311-319, XP008078769
- ADLER WÜRTZEN ET AL: INT ARCH ALLERGY IMMUNOL, vol. 144, 2007, pages 287-295,
- MARSH ET AL: IMMUNOLOGY, 1970, pages 705-722,

Description

TECHNICAL FIELD

The present invention relates to an in vitro method of evaluating the immunological activity, including the allergenic activity and potential for inducing allergic reactions, i. a. potential for inducing anaphylaxis, of a vaccine preparation in the form of a mixture of a molecular protein antigen and a solid phase carrier in the form of aluminium hydroxide, wherein the mixture comprises a liquid phase and a solid phase, to which at least a part of the antigen is attached.

BACKGROUND OF THE INVENTION

Vaccines for e.g. subcutaneous injection may be prepared by mixing an aqueous solution of an antigen and a solid phase carrier, e.g. aluminium hydroxide gel, to produce a mixture, wherein at least a part of the antigen is adsorbed to the solid phase and part of or none of the antigen is in the liquid phase. The solid phase carrier may serve as an adjuvant, i.e. it potentiates the immune response of the antigen, although the mechanism of the potentiation is not always fully understood. Also, the mechanism and nature of the adsorption of the antigen to the solid phase carrier is not always fully understood and may depend strongly on the type of antigen involved. Theoretically, however, the adsorption to aluminium hydroxide gels partly involves electrostatic forces. For proteins, it is believed that the phosphate groups of phosphorylated proteins also interact with the aluminium hydroxide gel and possibly to some extent replaces the hydroxide groups in the gel structure.

Consequently, the degree of adsorption varies with the nature of the specific protein in question. Also, in the case of an antigen in the form of an extract of a biological material, e.g. an extract of grass pollen allergens, the extract contains a number of different ions and molecules, which potentially interferes with the bonding of the allergens to the solid phase carrier.

The immunological activity may be measured in an in vivo method involving the administration of the vaccine to a test animal to raise antibodies to the antigen, collecting a biological sample and assaying the sample to measure the amount of antibodies raised. The allergenic activity and the potential for inducing allergic reactions may be tested by intradermal injection in sensitised animals, and by measurement of the extent of the wheat and flare reactions (Kildsgaard et al., Assessment of the in vivo allergenic potency of new allergy vaccines by intradermal testing in sensitised mice, Clinical Immunology and Allergy in Medicine, Proceedings of the 21st EAACI Congress 2002, Naples, Italy). However, such in vivo methods are laborious and time-consuming, and they necessitate the use of test animals, which is undesirable.

SU-A-1 746 318 discloses a method of quantitative determination of antigen in tick-borne encephalitis vaccine preparation, wherein the antigen is adsorbed on aluminium hydroxide, the method comprising reacting the vaccine preparation with an excess amount of specific antibodies in phosphate buffer, followed by immunoenzymatic determination of the amount of antibodies in the supernatant. A calibration graph is used to obtain quantitative results.

Chang et al. (Vaccine 19 (2001) 2884-2889) discloses a study examining the degree of lysozyme adsorption to aluminium hydroxide gel in the vaccine and in interstitial fluid and its effect on the immune response in rabbits. Vaccines were pre-treated with phosphate anion to produce vaccines having degrees of adsorption ranging from 3 to 90 %. It was found that the degree of adsorption of vaccines exhibiting 3, 35 or 85 % adsorption changed to 40 % within on hour of mixing with interstitial fluid to simulate subcutaneous administration. In accordance with this, the anti-lysozyme antibody response was the same for vaccines having different degrees of adsorption.

Shi et al. (Vaccine 20 (2002) 80-85) discloses a study of the ability of interstitial fluid to change the degree of adsorption of ovalbumin to aluminium hydroxide adjuvant and lysozyme to aluminium phosphate adjuvant. Ovalbumin and lysozyme were almost completely eluted after exposure at 37° C to lymph fluid. The ability of lymph fluid to elute lysozyme from aluminium phosphate adjuvant did not change as the vaccine aged. Only 60 % of the ovalbumin adsorbed to aluminium hydroxide was eluted during exposure to lymph fluid after the vaccine aged for 11 weeks at 4° C.

Iyer et al. (Vaccine 21 (2003) 1219-1223) discloses the finding that ovalbumin and dephoshorylated alpha casein were adsorbed in an aluminium hydroxide vaccine but were completely eluted when exposed to interstitial fluid. The vaccine nevertheless produced immunopotentiation compared to a solution of the protein. In contrast, alpha casein was completely adsorbed to aluminium hydroxide in both the vaccine and upon exposure to interstitial fluid. Immunopotentiation by aluminium hydroxide was also observed for alpha casein. The results indicated that antigen presenting cells can take up desorbed antigen from interstitial fluid as well as antigen adsorbed to aluminium-containing adjuvants.

Katz et al. (Journal of Virological Methods, 25 (1989) 101-108) discloses an ELISA for assessing the antigenic content of inactivated aluminium hydroxide adjuvanted virus vaccines. The ELISA is stated to supplement in vivo testing. Other in vitro methods comprise radioimmunoassay and methods requiring antigen desorption from aluminium hydroxide. Unlike such in vitro methods the ELISA did not suffer significant interference from the aluminium hydroxide except at high aluminium hydroxide concentrations. It is mentioned that previous attempts at an ELISA of intact vaccines suffered extreme inhibition by aluminium hydroxide. The article sets forth various explanations why the present ELISA works. In the ultimate paragraph it is suggested that the method may be applicable to aluminium hydroxide adjuvanted virus vaccines as a class.

Thraenhart et al. (Journal of Biological Standardisation, (1989) 17, 291-309) discloses an ELISA for in vitro potency testing of rabies virus vaccine by determination of rabies virus glycoprotein. The influence of aluminium hydroxide on the potency measurement was investigated and it was found that there was no influence.

US-A-4 127 385 (Weeke) describes a method comprising mixing allergen extracts of horsehair and scale adhered to an alhydrogel with serum from an allergic patient to bind the free IgE of the serum to the allergen of the alhydrogel and subsequently adding radiolabelled anti-IgE and measuring the radioactivity. It is indicated that the method can be used to determine the strength or the storage life of allergen extracts adhered to alhydrogel. This type of prior art immunoassay has the disadvantage that antibodies not specific for the allergen will to a certain extent bind to the alhydrogel-allergen and result in an incorrect measurement.

The nature of molecular antigen adsorption to the solid phase carriers is very complex and largely unknown, and also it varies among different antigens depending on the chemical and structural nature of the antigen. Furthermore, the influence of the solid phase carrier in the reaction between antigen-specific IgE and antigen bound to a solid phase carrier is very complex and not fully known. Therefore, it has until now been believed that it is not possible to measure in vitro the immunological activity, including the allergenic activity and potential for inducing allergic reactions, of ready-to-use solid phase carrier vaccines comprising molecular antigens, or at least that it is not possible to measure it accurately. Thus, up to now it has been common practise to evaluate the immunological activity of a vaccine in vitro on the basis of a measurement of the immunological activity of the solution of molecular antigen used for the preparation of the ready-to-use solid phase carrier vaccine. The object of the present invention is to provide an in vitro method of evaluating the immunological activity of ready-to-use, solid phase carrier, molecular protein antigen vaccines, wherein the solid phase carrier is in the form of aluminium hydroxide.

## SUMMARY OF THE INVENTION

This object is achieved with the present invention, which relates to the following aspects:

An in vitro method of evaluating the immunological activity of a vaccine preparation in the form of a mixture of a molecular protein antigen and a carrier in the form of aluminium hydroxide,

wherein the mixture comprises a liquid phase and a solid phase, to which at least a part of the antigen is attached, the method comprising the steps of

i) subjecting the vaccine to one or more measurements selected from the group consisting of:

1) the immunological activity of the mixture,
2) the immunological activity of antigen in the liquid phase,
3) the immunological activity of antigen in the solid phase,
4) the immunological activity of antigen in the liquid phase upon a treatment of the mixture to displace the antigen from the solid phase, and
5) the immunological activity of antigen in the solid phase upon a treatment of the mixture to displace the antigen from the solid phase,

wherein the immunological activity measurement is selected from the group consisting of a) antibody binding capacity using an immunoassay employing an antigen-specific antibody bound to an antibody solid phase, b) ability to activate effector cells and c) potential for inducing anaphylaxis; and
ii) using the measurement results to evaluate the immunological activity of the vaccine.

A method of preparing a vaccine preparation in the form of a mixture of a molecular protein antigen and a solid phase carrier in the form of aluminium hydroxide, wherein the mixture comprises a liquid phase and a solid phase, to which at least a part of the antigen is attached, the method comprising

i) mixing the antigen and the carrier,
ii) measuring the immunological activity of the vaccine using the method of evaluating the immunological activity of a vaccine preparation according to the invention, and
iii) repeating steps i) and ii) until a desired immunological activity is obtained.

The present invention is based on the novel and surprising finding that it is indeed possible to perform measurements of the immunological activity, including the allergenic activity and potential for inducing allergic reactions, i. a. potential for inducing anaphylaxis, on ready-to-use solid phase carrier, e.g. gel, vaccines, using e.g. conventional competitive immunoassays, histamine release assays and T cell proliferation assays, without the solid phase carrier prevents valid and meaningful measurements to be made. In particular, the present invention is based on the novel and surprising finding that when the immunological activity is measured as the antibody binding capacity, it is possible to avoid the disturbing influence on the measurement by the solid phase carrier by using antibody bound to an antibody solid phase. It is believed that the reason for this is that the antibody solid phase prevents unspecific binding of antibody to the antigen-solid phase carrier system. This finding is surprising, since it might well be expected that it would be difficult for antibodies coupled to an antibody solid phase to obtain contact with the antigens of an antigen-solid phase carrier system. The same considerations apply to the situation, where the immunological activity is measured as the ability to activate effector cells, wherein the antigen-specific antibodies are bound to the effector cells, e.g. mast cells and basophils, which resemble a particulate antibody solid phase.

The invention is further based on the recognition that the problem of the complexity and uncertainties of the nature of antigen adsorption to solid phase carriers and the variability from one type of antigen to another can be eliminated 1) by comparing the measurement results obtained with historical results from the same type of antigen, 2) by relating measurement results for stored vaccines with results for the freshly prepared vaccine, and/or 3) by obtaining a detailed characterisation of the vaccine by measuring a number of characteristic parameters of the vaccine, including the distribution of antigen between the liquid phase and the solid phase and the strength of the adsorption of the antigen to the solid phase carrier.

## SHORT DESCRIPTION OF THE FIGURES

Fig. 1 shows the ability of Phl p extract adsorbed to an aluminium hydroxide gel (Alutard Phl p) to inhibit the binding of IgE to biotinylated Phl p extract using Phl p extract in solution (Phl p) as reference and Bet v extract (Alutard Bv) as negative control.

Fig. 2 shows histamine release levels of a vaccine in the form of Phl p extract allergen adsorbed to aluminium hydroxide gel adjuvant (Vaccine A), of the supernatant of Vaccine A after sedimentation, and of the solid phase of Vaccine A after sedimentation.

Fig. 3 shows the histamine release levels of a vaccine in the form of Phl p extract allergen adsorbed to aluminium hydroxide gel adjuvant (+alum) and of Phl p extract allergen in solution (-alum).

Fig. 4 shows T cell stimulation using CD69 as marker for four Phl p extract aluminium gels, the supernatants thereof, for two Phl p extracts in solution and for purified Phl p 1 and Phl p 5 in solution.

Fig. 5-6 shows the displacement (desorption) of allergen from aluminium hydroxide gel vaccines (Alutard) comprising the allergens Phl p 1 and Phl p 5, respectively.

## DETAILED DESCRIPTION OF THE INVENTION

### Antigen

In connection with the present invention "antigen" means any immunogenic substance, i.e. any substance capable of activating the immune system.

In connection with the present invention "molecular protein antigen" means any substance in the form of a single molecule or a mixture of single molecules, wherein the single molecules are proteins, as well as analogues and derivatives thereof. The expression "molecular antigen" excludes vira and microbial cells, such as bacterial and fungal cells.

The molecular protein antigen may i.a. be selected from the group consisting of proteinaceous allergens, proteinaceous medicaments, and proteinaceous nutritional substances, as well as analogues or derivatives thereof.

Examples of molecular protein antigens are proteinaceous allergens, peptides, as well as analogues or derivatives thereof. Examples of proteinaceous nutritional substances are proteinaceous enzymes, as well as analogues or derivatives thereof. Examples of proteinaceous medicaments are antibodies, peptides, proteinaceous hormones, antibodies, peptides, proteinaceous hormones, proteinaceous enzymes, as well as analogues or derivatives thereof.

In the present context, the term analogues or derivatives is intended to include modified forms of the biologically active substance. The modification can be made by chemical modification or synthetic modification, e.g. by biotinylation, deamination, maleination, substitution of one or more amino acids, by cross-linking, by glycosylation, or by other recombinant technology. The term is also intended to include natural-occurring mutations, isoforms and retroinverse analogues.

The molecular protein antigen may preferably be selected from the group consisting of:

proteinaceous enzymes such as urokinase, TPA (tissue plasminogen activator), coagulation Factor VIII, and streptokinase;

proteinaceous immunogenic substances such as natural, recombinant or modified proteins or fragments thereof, proteinaceous antigens, proteinaceous allergens (cf. below), peptides, molecular protein components of parasitic material or proteinaceous toxins, e.g. such derived from

Tetanus toxoid, Diphtheria toxoid, Cholera toxin A and B subunits, Rubella, Rhabdovirus (rabies), Myoxoviruses, Paramyoxyviruses like parainfluenza virus, mumps and meales, Picomaviruses like poliovirus, coxsackievirus, echovirus and rhinovirus, Reoviruses, Poxviruses like small pox virus, Vaccinia virus and cowpox virus, Papovaviruses like polyoma virus, papilloma virus and SV-40, Adenoviruses, EBV like mononucleosis virus, Parvoviruses like HPV B19, Herpes viruses like Herpes simplex virus, and Herpes zoster virus (Varicella virus), Cytomegalovirus (CMV), Arboviruses like yellow fever and Dengue fever, Retroviruses like HIV, Hepatitis viruses like Hepatitis A, Hepatitis B and Hepatitis C, Haemophilius influenzae type B, Mycobacterium like M. tuberculosis, M. bovis, M. africanum, M. microti, M. avium, M. intracellulare, M. karisasii, M. gordonae, M. paratuberculosis, and M. lepramurium, Borrelia spp. like B. burgdorferi, in particular B. burgdorferi sensu lato and B. burgdorferi sensu stricto, B. garinii, B. afzelii, B. duttoni and B. recurrentis, Bordetella pertussis (whooping cough), Salmonella spp. like S. typhimurium and S. typhi, Treponema spp. like T. pallidum, Leptospira spp., Campylobacter spp. like C. jejuni, Helicobacter spp. like H. pylori, Pseudomonas spp., Legionella spp., Neisseria spp. like N. gonorrhoea and N. menigitidis, Chlamydia spp. like C. trachomatis, C. pneumonia and C. psittae, Enterobacter spp., Klebsiella spp., Yersinia spp., Vibrio spp. like Vibrio cholerae, Gardnerella spp., Rickettsia spp., Clostridium spp. like C. difficile, C. botulinum and C. tetani, Lactobacillus spp., Listeria spp., and Mycoplasma spp. like M. pneumoniae M. hominis, Plasmodium falciparum, and Leishmania donovani,

moulds and fungi such as Clahdosporium, Alternaria, Aspergillus, Besidiomycetes, Candida albicans, and Penicillinum;

proteinaceous medicaments such as

proteinaceous enzymes,

proteinaceous hormones e.g. LHRH, insulin and human growth hormone. proteinaceous hemolytics/hemostatics e.g. erythropoetin $\alpha$ or $\beta$,

other proteinaceous medicaments like cancer-related compounds, such as TNF$\alpha$, LHRH analogues, cytostatica, and anti-cancer antibodies, such as antibodies against breast cancer cells, e.g. antibodies against HER-2 receptor, colon cancer cells, and B cell lymphoma cells, e.g. antibodies against CD20 on malign B cells;

as well as analogues or derivatives thereof.

In a preferred embodiment of the invention the molecular protein antigen is an allergen. In a preferred embodiment of the invention the allergen is any naturally occurring protein that has been reported to induce allergic, i.e. IgE mediated reactions upon their repeated exposure to an individual. Examples of naturally occurring allergens include pollen allergens (tree-, herb, weed-, and grass pollen allergens), insect allergens (inhalant, saliva and venom allergens, e.g. mite allergens, cockroach and midges allergens, hymenopthera venom allergens), animal hair and dandruff allergens (from e.g. dog, cat, horse, rat, mouse etc.), and food allergens. Important pollen allergens from trees, grasses and herbs are such originating from the taxonomic orders of Fagales, Oleales, Pinales and platanaceae including i.a. birch (Betula), alder (Alnus), hazel (Corylus), hornbeam (Carpinus) and olive (Olea), cedar (Cryptomeria and Juniperus), Plane tree (Platanus), the order of Poales including i.a. grasses of the genera Lolium, Phleum, Poa, Cynodon, Dactylis, Holcus, Phalaris, Secale, and Sorghum, the orders of Asterales and Urticales including i.a. herbs of the genera Ambrosia, Artemisia, and Parietaria . Other important inhalation allergens are those from house dust mites of the genus Dermatophagoides and Euroglyphus, storage mite e.g Lepidoglyphys, Glycyphagus and Tyrophagus, those from cockroaches, midges and fleas e.g. Blatella, Periplaneta, Chironomus and Ctenocepphalides, and those from mammals such as cat, dog and horse, venom allergens including such originating from stinging or biting insects such as those from the taxonomic order of Hymenoptera including bees (superfamily Apidae), wasps (superfamily Vespidea), and ants (superfamily Formicoidae). Important inhalation allergens from fungi are i.a. such originating from the genera Alternaria and Cladosporium.
In a more preferred embodiment of the invention the allergen is Bet v 1, Aln g 1, Cor a 1 and Car b 1, Que a 1, Cry j 1 ,Cry j 2, Cup a 1 ,Cup s 1 ,Jun a 1, Jun a 2, jun a 3, Ole e 1, Lig v 1, Pla l 1, Pla a 2, Amb a 1, Amb a 2, Amb t 5, Art v 1. Art v 2 Par j 1, Par j 2 ,Par j 3 ,Sal k 1, Ave e 1, Cyn d 1, Cyn d 7, Dac g 1, Fes p 1, Hol l1, Lol p 1 and 5, Pha a 1, Pas n 1, Phl p 1, Phl p 5, Phl p 6, Poa p 1, Poa p 5, Sec c 1, Sec c 5, Sor h 1, Der f 1, Der f 2, Der p 1, Der p 2, , Der p 7, Der m 1, Eur m 2, Gly d 1, Lep d 2, Blo t 1, Tyr p 2, Bla g 1, Bla g 2, Per a 1, Fel d 1, Can f 1, Can f 2 , Bos d 2, Equ c 1, Equ c 2, Equ c 3 , Mus m 1, Rat n 1, Apis m 1, Api m 2 , Ves v 1, Ves v 2, Ves v 5, Dol m 1, Dil m 2, Dol m 5, Pol a 1, Pol a 2, Pol a 5, Sol i 1, Sol i 2, Sol i 3 and Sol i 4, Alt a 1, Cla h 1, Asp f 1, Bos d 4, Mal d 1, Gly m 1, Gly m 2, Gly m 3, Ara h 1, Ara h 2, Ara h 3, Ara h 4, Ara h 5 or shufflant hybrids from Molecular Breeding of any of these.

In the most preferred embodiment of the invention the allergen is grass pollen allergen or a dust mite allergen or a ragweed allergen or a cedar pollen or a cat allergen or birch allergen.

In yet another embodiment of the invention the vaccine comprises at least two different species of allergens either originating from the same allergic source or originating from different allergenic sources e.g. grass group 1 and grass group 5 allergens or mite group 1 and group 2 allergens from different mite and grass species respectively, weed antigens like short and giant ragweed allergens, different fungis allergens like alternaria and cladosporium, tree allergens like birch, hazel, hornbeam, oak and alder allergens, food allergens like peanut, soybean and milk allergens .

The allergen incorporated into the vaccine may be in the form of an extract, a purified allergen, a modified allergen, a recombinant allergen or a mutant of a recombinant allergen. An allergenic extract may naturally contain one or more isoforms of the same allergen, whereas a recombinant allergen typically only represents one isoform of an allergen. In a preferred embodiment the allergen is in the form of an extract. Preferably, the immunological activity of two or more major and/or minor allergens of the extract is measured. In addition the immunological activity of the whole extract may be measured.

In another preferred embodiment the allergen is a recombinant allergen. In a further preferred embodiment the allergen is a naturally occurring low IgE-binding mutant or a recombinant low IgE-binding mutant.

Allergens may be present in equi-molar amounts or the ratio of the allergens present may be in the range of from 1:1 to 1:40, preferably from 1:1 to 1:20 and more preferably from 1:1 to 1:10.

In a further embodiment of the invention the low IgE binding allergen is an allergen according to WO 99/47680 or WO02/40676.

### Solid phase carrier

The solid phase carrier is in the form of aluminium hydroxide.

### Displacement of antigen from the solid phase

In one embodiment of the invention, the displacing treatment comprises contacting the mixture with a protein-containing reagent. It is believed that the displacing potential increases with increasing electrochemical charge of the protein, and hence charged proteins are preferred. Any protein may be used to effect displacement of antigen from the solid phase carrier. Preferred protein-containing reagents are body fluids, such as lymph fluid, interstitial fluid, blood plasma, blood serum, purified fractions of body fluids and proteins isolated form body fluids, such as Human Serum Albumin (HAS). Preferably, the body fluid is blood serum. The use of body fluids for the displacement of antigen from the solid phase carrier makes it possible to simulate the in vivo conditions, which the vaccine is subjected to after administration.

In a second embodiment, the displacing treatment comprises contacting the mixture with anions, such as phosphate ions, citrate ions, lactate ions, acetate ions, sulphate ions, borate ions and oxalate ions, preferably, phosphate ions.

Preferably, the displacement is carried out at a pH of from 4 to 10, more preferably from 5 to 9, and most preferably from 6 to 8. Preferably, the displacement is carried out at a temperature of from 32 °C to 42 °C, more preferably from 35 °C to 39 °C, and most preferably from 36 °C to 38 °C.

### Measurement of antibody binding capacity

In connection with the present invention the expression "antibody binding capacity" means the level of B cell epitopes available in the vaccine for antibody binding. The measurement of antibody binding capacity of the vaccine preparation may be carried out using any suitable method or immunoassay capable of performing such a measurement, wherein the antibody is bound to an antibody solid phase. Suitable types of assays include 1) assays wherein the antigen to be assayed is passively attached to a solid phase, and 2) assays wherein the antigen to be assayed is captured by a first antigen-specific antibody coupled to a solid phase. For both type 1) and 2) assays, the antigen attached to the solid phase may a) be reacted with a second antigen-specific antibody, or b) with a modified antigen.

When using option a), i) the second antigen-specific antibody may be labelled (direct assay) or ii) it may be reacted with a labelled anti-antibody specific to the second antigen-specific antibody (indirect assay). When using option b), the modified antigen may be labelled or be adapted to be coupled to a label, e.g. by a linker system. One example of such a linker system is the biotin-avidin/streptavidin system.

The label may be any suitable label system conventionally used in immunoassays comprising chromogenic labels, luminescent labels, chemiluminescent labels, enzyme labels, radioactivity labels, fluorescent labels, and absorbance labels, preferably chemiluminescent labels.

In a preferred embodiment of the invention, the (iv) label compound is a chemiluminescent compound covalently bound to avidin, streptavidin or a functional derivative thereof.

The chemiluminescent label is preferably an acridinium compound, such as dimethylacridiniumester (DMAE).

The first and second antigen-specific antibody and the anti-antibody may all independently of each other be either monoclonal or polyclonal.

The assay of type 2)a) is commonly referred to as a sandwich assay or a two-site assay. The assay of type b) is commonly referred to as an inhibition assay, when the antigen to be assayed is allowed to become attached to the solid phase prior to adding to modified antigen. The assay of type b) is commonly referred to as a competition assay, when the antigen to be assayed and the modified antigen are mixed prior to becoming attached to the solid phase.

In a preferred embodiment of the present invention, the immunoassay is a competitive assay or an inhibition assay, preferably a competitive assay.

In a preferred type of competitive immunoassay the immunological activity of a vaccine is measured as the degree of inhibition of the bonding between standardised biotinylated antigen and antigen-specific IgE by the antigen-containing vaccine. The immunoassay comprises the steps of 1) mixing the antigen-containing vaccine preparation with biotinylated antigen to form an antigen mixture, 2) incubating the antigen mixture with antigen-specific IgE coupled to an antibody solid phase, e. g. a particulate carrier, such as paramagnetic particles, to form an immunocomplex, and 3) optionally washing and subsequently incubating the immunocomplex with streptavidin labelled with acridinium ester, and 4) washing and subsequently measuring the amount of light emitted. The immunoassay may be carried out using e.g. an ADVIA Centaur (Bayer).

Examples of suitable immunoassays are ELISA-based assays and RAST.

In a further suitable immunoassay for carrying out the method of the invention, 1) a quantified amount of antigen-specific antibody is reacted with the antigen vaccine to be assayed, 2) in the resulting reaction mixture, the liquid phase is separated from the solid phase, and 3) the remaining amount of unbound antibody in the liquid phase is measured. The measurement of antibody in the liquid phase may be carried out using any conventional method for quantifying antibody. In a variant of this immunoassay, the liquid phase is not separated from the solid phase before the measurement of unbound antibody.

The type of antibody used or detected in the immunoassay determines the type of epitopes measured. Thus, dependent on the type of antibody, e.g. IgA, IgE, IgG and IgM, used or detected it is possible to selectively measure IgA, IgE, IgG and IgM epitopes, respectively. In a preferred embodiment of the invention, the antibody used or detected is selected from the group consisting of IgA, IgE, IgG, IgM and combinations thereof. In a particular embodiment of the invention, the antibodies used or detected are both IgE and IgG. In a preferred embodiment of the invention, the antibody used or detected is IgE.

**Antibody solid phase**

The antibody solid phase may be any solid phase conventionally used in immunoassays, including microtiter plates and particles, e.g. paramagnetic particles.

**Measurement of ability to activate effector cells**

In a preferred embodiment of the method of the invention, the immunological activity is measured as the ability to activate effector cells of the immune system.

In one embodiment of the invention, whole blood is used for effector cell activation. In a second embodiment, the effector cells are cells isolated from a biological sample. In a third embodiment, the effector cells are cells isolated from a biological sample and cultivated. In a fourth embodiment, the effector cells are cells isolated from a biological sample, cultivated and modified, e.g. genetically modified.

Preferably, the effector cells are selected from the group consisting of mast cells, basophils, eosinophils, T cells, B cells and Antigen Presenting Cells (APC), and combinations thereof. Other preferred effector cells are modified effector cells, i.e. cells derived from and having at least some features of effector cells, including genetically modified cells and malignantly transformed cells.

In one embodiment of the invention, the effector cell activating ability is measured by measuring the level of an effector cell marker. The marker is preferably selected from the group consisting of secretory molecules, surface molecules and intracellular molecules. Preferably, the secretory molecule is selected from the group consisting of mediators, cytokines, cytotoxic proteins and soluble receptors.

Examples of the mediator to be measured are mediators selected from the group consisting of histamine, leucotrienes (LTB$_4$, LTC$_4$, LTD$_4$ and LTE$_4$), prostaglandines(PGD$_2$, PGE$_2$ and PGF$_{2a}$), thromboxane, Platelet Activating Factor (PAF), Major Basic Protein (MBP), Eosinophil Cationic Protein (ECP), Eosinophil Derived Neurotoxin (EDN), Eosinophil Peroxidase (EPO), bradykinin, adenosine, Substance P, Neurokinin A, complement factors (e.g. C3d), including complement fragments; Serotonin, Oxygen Radicals, basogranulin, and mast cell and basophil proteases, including tryptase, chymase, carboxypeptidase and cathepsin.

Examples of the cytokine to be measured are cytokines selected from the group consisting of Interleukins (IL-1 to IL-27), hematopoietric growth factors, granulocyte-macrophage colony stimulating factors (e.g. CM-CSF), interferons (IFN$\alpha$, IFN$\beta$, IFN$\gamma$), TNF, lymphotoxin, immunoglobulin-superfamily members (Intercellular Adhesion Molecule-1 (ICAM-1), Vascular Cell Adhesion Molecule-1 (VCAM-1)), the TGF-beta family and the chemokines (IL-8, RANTES). Assays for measuring the following cytokines are widespread: IL-2, IL-4, IL-5, IL-6, IL-10, IL-12, IL-13, IFN-gamma, TNF-alpha, TGF-beta.

Examples of the cytotoxic protein to be measured are cytotoxic proteins selected from the group consisting of Eosinophil Cationic Protein (ECP), Major Basic Protein (MBP) and Eosinophil Derived Neurotoxin (EDN).

Preferably, the surface molecule is selected from the group consisting of surface receptors and adhesion molecules, such as selectins, integrins and immunoglobulin superfamily members (Intercellular Adhesion Molecule-1 (ICAM-1), Vascular Cell Adhesion Molecule-1 (VCAM-1)), Very Late Activation Antigen-4 (VLA4), CD11 B, CD11C, and CD18. Surface molecules known to be up- or downregulated in effector cells by antigen activation are CD23, CD69, CD203C , CD31, CD162 and CD162L. Other surface molecules include basogranulin.

Preferably, the effector cell marker is histamine, tryptase, basogranulin, leucotrien LTC$_4$, CD63, CD69 and CD203C. Histamine may e.g. be measured in an ELISA-based method based on the competition between histamine to be assayed and its enzyme conjugate, histamine-alkaline phosphatase used as tracer for binding to antibody coated onto microwells. The monoamine histamine is too small to occupy completely the binding site on the antibody. High affinity monoclonal antibodies directed against modified histamine have therefore been obtained. The histamine in the sample must be derivatized in the same manner as the histamine of the conjugate. This is achieved readily and reproducibly with an acylating reagent at slightly alkaline pH. The acylated histamine of the sample, and the histamine-alkaline phosphatase conjugate, when added to the microtiter wells, compete for binding to a limiting number of antibody sites. After incubation, the wells are rinsed in order to remove non-bound components. The bound enzymatic activity is then measured by the addition of a chromogenic substrate (pNPP). The intensity of the color depends inversely on the concentration of histamine in the sample. The concentration is calculated on the basis of a standard curve obtained with standards. This enzyme immunoassay may be carried out using a kit obtainable from "IMMUNOTECH" (Marseille, France).

In a second embodiment of the invention, the effector cell activating ability is measured by measuring the T cell proliferation. The T cell proliferation may be measured by a method based on incorporation of 3H-thymidine or the reduction of fluorescence labelling and may be conducted using freshly isolated leucocytes from the blood of sensitized subjects or using established allergen-specific T-cell lines. In addition, the cytokine production of the activated cells may be investigated by analysis of the cell supernatants by ELISA or beads based methods.

Early events in the T-cell activation may be investigated through flow cytometric analysis of T-cell expression of different surface receptors such as CD25, 26, 27, 39, 45 RA/O, 69, 70, 96, 97, 108, 109, 134 (OX40), 153, 154 (OX40L), 166, 178 (FasL), 183 (CXCR3), 212 (IL-12Rb1), 223, which are up-or down-regulated at different time-points during T-cell activation.

The activation of T-cells may be influenced by the differentiation and activation stage of the antigen presenting cells (APC), which may be investigated by flow cytometric analysis of the following surface molecules: CD14, 25, 26, 40, 80/86, 83, 105, 166.

Finally, the immunological effect of the vaccines on B-cell activation may be described via the surface expression of CD25, 26, 39, 80/86, 97, 126, 138 and surface expression as well as secretion of different antibody isotypes. As an additional alternative the majority of the parameters described above may be investigated at the mRNA level through Taqman analysis, gene chip analysis, or other method for quantifying gene expression.

Measurement of potential for inducing anaphylaxis

Administration of a vaccine involves a certain level of risk of eliciting IgE mediated side effects, e.g. anaphylaxis. In a particular aspect of the method of the invention, the immunological activity of the vaccine preparation is measured as the potential for inducing anaphylaxis. Preferably, the potential for inducing anaphylaxis is measured by measuring the level of an effector cell anaphylaxis marker selected from the group consisting of effector cell markers mentioned above. Preferably, the effector cell anaphylaxis marker is histamine, tryptase, basogranulin, leucotrien LTC4, CD63, CD69 and CD203C. Histamine is released from mast cells and basophils. Histamine may e.g. be measured in the ELISA-based method described above. Also, histamine may be measured using glass fibre based assays.

In a preferred embodiment of this aspect of the invention, the potential for inducing anaphylaxis in whole blood is measured. Preferably, the whole blood used for the measurement has been withdrawn from a subject maximally five hours, more preferably two hours, prior to the measurement.

It is preferred that the vaccine and the whole blood is mixed in a ratio corresponding to a calculated in vivo ratio, which would occur in a subject as a result of an accidental administration of a vaccine dose into the blood stream of a subject. Also, it is preferred that the whole blood adjusted to body temperature is used.

In an alternative embodiment of this invention, the potential for inducing anaphylaxis 1) in effector cells isolated from a biological sample, 2) in effector cells isolated from a biological sample and cultivated, or 3) in effector cells isolated from a biological sample, cultivated and modified, e.g. genetically modified, is measured.

Preferably, the effector cells are selected from the group consisting of mast cells, basophils, eosinophils, T cells, B cells and Antigen Presenting Cells (APC), and combinations thereof. Other preferred effector cells are modified effector cells, i.e. cells derived from and having at least some features of effector cells, including genetically modified cells and malignantly transformed cells. Genetically modified cells may e.g. be cells genetically modified so as to express one or more proteins, which are not expressed in native cells, including intracellular proteins and surface proteins, e.g. receptor proteins. Malignantly transformed cells may e.g. be a cancer cell line, e.g. a cancer cell capable of continuous in vitro growth without stimulation.

### Immunological activity measurements 1)-5)

In one embodiment of the invention, the vaccine is subjected solely to a measurement of the immunological activity of the mixture of the liquid phase and the solid phase (measurement 1)).

In a second embodiment of the invention, the vaccine is subjected solely to a measurement of the immunological activity of antigen in the liquid phase upon a treatment of the mixture to displace the antigen from the solid phase (measurement 4)).

In a third embodiment of the invention, the vaccine is subjected both to a measurement of the immunological activity of the mixture of the liquid phase and the solid phase (measurement 1)), and to a measurement of the immunological activity of antigen in the liquid phase (measurement 2)).

In a fourth embodiment of the invention, the vaccine is subjected both to a measurement of the immunological activity of antigen in the liquid phase (measurement 2)), and to a measurement of the immunological activity of antigen in the solid phase (measurement 3)).

In a fifth embodiment of the invention, the vaccine is subjected both to a measurement of the immunological activity of the mixture of the liquid phase and the solid phase (measurement 1)), and to a measurement of the immunological activity of antigen in the liquid phase upon a treatment of the mixture to displace the antigen from the solid phase (measurement 4)).

### Evaluation of immunological activity of vaccine

A preferred embodiment of the method of the invention is one, wherein the immunological activity, including allergenic activity and potential for inducing allergic reactions, e.g. potential for inducing anaphylaxis, of an antigen-containing intermediate product used for preparing the vaccine is measured, and wherein the evaluation of the immunological activity of the vaccine is based on a comparison of the measurement result obtained for the intermediate product and the measurement results obtained for one or more of measurements 1) - 5). Preferably, the vaccine is subjected to the measurements immediately upon preparation.

A further preferred embodiment of the invention is one, wherein the vaccine is subjected to the measurements immediately after preparation and after one or more periods of storage, and wherein the evaluation of the immunological activity of the vaccine is based on a comparison between the former and latter measurement results.

Yet a further embodiment of the invention is one, wherein the evaluation of the immunological activity of the vaccine is based on a comparison between the measurement results obtained for the vaccine and prior measurement results from the same type of vaccine or from another type of vaccine, wherein the same type of immunological activity measurement is used to obtain the measurement results for the vaccine and the prior measurement results.

### Vaccines

The vaccine preparation subjected to the method of the present invention may be any ready-to-use preparation in the form of a mixture of a molecular protein antigen and a solid phase carrier in the form of aluminium hydroxide, wherein the mixture comprises a liquid phase and a solid phase, to which a part of the antigen is attached, or any such preparation for preparing a ready-to-use formulation.

The ready-to-use preparation may be for parenteral administration and for mucosomal administration.

Parenteral administration includes intravenous, intramuscular, intraarticular, subcutaneous, intradermal, epicutaneous/transdermal and intraperitoneal administration. Vaccines for administration via injection may be formulated so as to be suitable for injection by needle or for needleless injection.

Mucosomal administration includes oral, nasal, vaginal, sublingual, ocular, rectal, urinal, intramammal, pulmonal, otolar (i.e. via the ear) or buccal administration.

The vaccine may be in the form of a spray, an aerosol, a mixture, a suspension, a dispersion, an emulsion, a gel, a paste, a syrup, a cream, an ointment, implants (ear, eye, skin, nose, rectal, and vaginal), intramammary preparations, vagitories, suppositories, or uteritories.

## Method of preparing a vaccine

The present invention further relates to a method of preparing a vaccine preparation in the form of a mixture of a molecular protein antigen and a solid phase carrier in the form of aluminium hydroxide, wherein the mixture comprises a liquid phase and a solid phase, to which at least a part of the antigen is attached, the method comprising

i) mixing the antigen and the carrier,
ii) measuring the immunological activity of the vaccine using the method according to the invention, and
iii) optionally repeating steps i) and ii) until a desired immunological activity is obtained.

## DEFINITIONS

The expression "in vitro method" means a method, which may be carried out without immunisations of test animals.

The expression "immunological activity" means any response of the immune system, including allergenic activity and potential for inducing allergic reactions, including potential for inducing anaphylaxis.

The expression "allergenic activity" means IgE binding activity.

The expression "solid phase carrier" means any water-insoluble substance capable of forming a covalent and/or a non-covalent attachment with an antigen, wherein the non-covalent attachment includes e.g. adherence, inclusion, encapsulation and coupling.

The expressions "solid phase" and "liquid phase" of a vaccine mean the phases resulting from a separation process for the separation of a suspension of the solid phase carrier in the liquid into a solid phase and a liquid phase, the separation process being e.g. centrifugation, extraction or simple sedimentation.

The expression "attached" means any covalent and/or a non-covalent attachment, wherein the non-covalent attachment includes e.g. adherence, inclusion, encapsulation and coupling.

## METHODS AND MATERIALS

### Preparation of aluminium gel adjuvant allergen vaccines

Lyophilised allergen is dissolved in an aqueous buffer and diluted to a desired concentration. "Alhydrogel" (1,3 %) is added to the allergen solution obtained wile stirring, and then sterile water is added. The resulting solution is allowed to stand to the following day, and then buffer is added slowly while stirring to produce the final allergen aluminium hydroxide gel.

## EXAMPLES

### Example 1

### IgE inhibition assay for allergen in solution and for allergen adsorbed to an aluminium hydroxide gel adjuvant

Method

IgE inhibition experiments were performed on an ADVIA centaur instrument. Serial dilutions (performed with the TECAN (P-05-07F294)) of the inhibitor (Antigen in solution or antigen gel adjuvant vaccine) were mixed with a fixed amount of biotinylated antigen and further incubated with a solid phase absorbed IgE. The amount of biotinylated allergen bound to the solid phase was estimated as the light emitted after incubation with streptavidin labelled with acridinium ester. The raw data was processed in Excel and transferred to GraphPad Prism v. 4.0 for the final analysis (curve fitting, plotting and statistical comparisons). The data was fitted to a four parameter logistic function (Eqn. 1),

$$Y = B + \frac{T - B}{1 + 10^{(\log_{10} EC50 - \log_{10} X) \cdot HillSlope}} \qquad (1)$$

and fitted curves was considered parallel if the HillSlope (HS) of the individual fits did not differ significantly. EC50 was estimated from fits constrained with a common HS estimate. EC50 denotes the concentration resulting in 50% inhibition.

Results

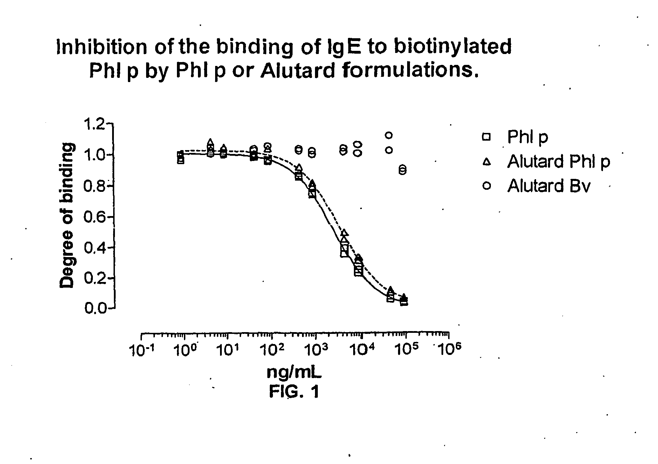

The IgE inhibition assay described above was used for testing the immunological activity of Phl p allergen and Bet v allergen gel adjuvant vaccines. The results are shown in Fig. 1.

Fig. 1 shows the ability of Phl p extract adsorbed to an aluminium hydroxide gel (Alutard Phl p) to inhibit the binding of IgE to biotinylated Phl p extract using Phl p extract in solution (Phl p) as reference and Bet v extract (Alutard Bv) as negative control.

The allergen extract in solution was the extract used for preparing the gel adjuvant vaccine and was used for comparison purposes.

From Fig. 1 the following may be concluded: As the maximum inhibiting potential of the Phl p extract gel corresponds to that of Phl p extract in solution, it can be concluded that the present IgE inhibition immunoassay is capable of measuring the full level and the full scope of specificity of immunological activity of the Phl p extract gel. The negative control Bet v extract gel shows no inhibitory activity.

As will appear from Fig. 1, the inhibition curve of the allergen gel vaccine is shifted somewhat to the right, the course of the shifted curves being parallel to that of the allergen in solution. This means that a higher concentration of the gel formulated allergen is needed to obtain the same degree of inhibition.

## Example 2

### Histamine release assay for allergen adsorbed to an aluminium hydroxide gel adjuvant

Method

Histamine was measured in an ELISA-based method based on the competition between histamine to be assayed and its enzyme conjugate, histamine-alkaline phosphatase used as tracer for binding to antibody coated onto microwells. The monoamine histamine is too small to occupy completely the binding site on the antibody. High affinity monoclonal antibodies directed against modified histamine have therefore been obtained. The histamine in the sample must be derivatized in the same manner as the histamine of the conjugate. This is achieved readily and reproducibly with an acylating reagent at slightly alkaline pH. The acylated histamine of the sample, and the histamine-alkaline phosphatase conjugate, when added to the microtiter wells, compete for binding to a limiting number of antibody sites. After incubation, the wells are rinsed in order to remove non-bound components. The bound enzymatic activity is then measured by the addition of a chromogenic substrate (pNPP). The intensity of the color depends inversely on the concentration of histamine in the sample. The concentration is calculated on the basis of a standard curve obtained with standards. The enzyme immunoassay was carried out using a kit obtainable from "IMMUNOTECH" (Marseille, France).

*Purpose:*

Histamine release with aluminium hydroxide formulated allergen in conditions resembling in vivo conditions in the event that vaccine is accidentally given in the blood stream. This is accomplished by measuring histamine release in undiluted whole blood to which vaccine is added. After stimulation the cells are spun down, and supernatant containing histamine is separated off. Then the histamine concentration is measured using Immunotech ELISA kit 2015.

*Reagents and materials:*

Pipes buffer pH 7.4 BB LAB97350
14 ml Falcon PP tubes
Venojekt heparin stabilised blood glass VT-100SH
PP Polypropylene Round bottom culture test tube: Elkay 12x75 mm 0002053001
PP stoppers
Immunotech ELISA kit 2015
ELISA washer ELP-40
ELISA reader EL-340 with KC4 program
Centrifuge with rotor for plates: Sigma 3-15
Incubator 37 °C
Coca 0.5 %
Shaking table Titramax 1000
Various single-, 8- and 12-channel pipettes

*Release:*

Heparin stabilised whole blood was pre-heated to 37 °C. Aliquots of various vaccines and controls were diluted 1:200 in Coca buffer. 2 $\mu$l were pippeted into Falcon tubes and diluted 1:500 in freshly drawn, undiluted whole blood. Incubation was performed at 37 °C for 30 min.

*Centrifugation:*

The tubes were centrifuged for 10 min. at 800 x g. The supernatants were collected and assayed using the histamine ELISA histamine.

*ELISA determination:*

1. Acetylation of samples and standards:

   Standards are pippeted into the plates with samples
   + 25 $\mu$l acetylation buffer is added by pippetes
   + 25 $\mu$l acetylation reagent

2. Thorough mixing with 12 channel pipette and 50 $\mu$l is transferred to precoated ELISA plate.
3. 200 $\mu$l Histamine conjugate pr. well is added
4. Incubation at 2-8 °C in refrigerator

   either a: on shaking table > 2 timer
   or b: without shaking > 18 timer

5. Washing on ELISA washer program: " Bot-wa-no-res 12" with the above washing buffer.
6. 200 $\mu$l color substrate pr. well is added
7. Incubation at 18-25°C (covered) on shaking table in 30 min.
8. Reaction stopped by adding 50 $\mu$l Stop solution.
9. Reading on Reader with KC4 at 405 nm.
10. Concentration is calculated in KC4 by linear fitting of standard curve.

Results

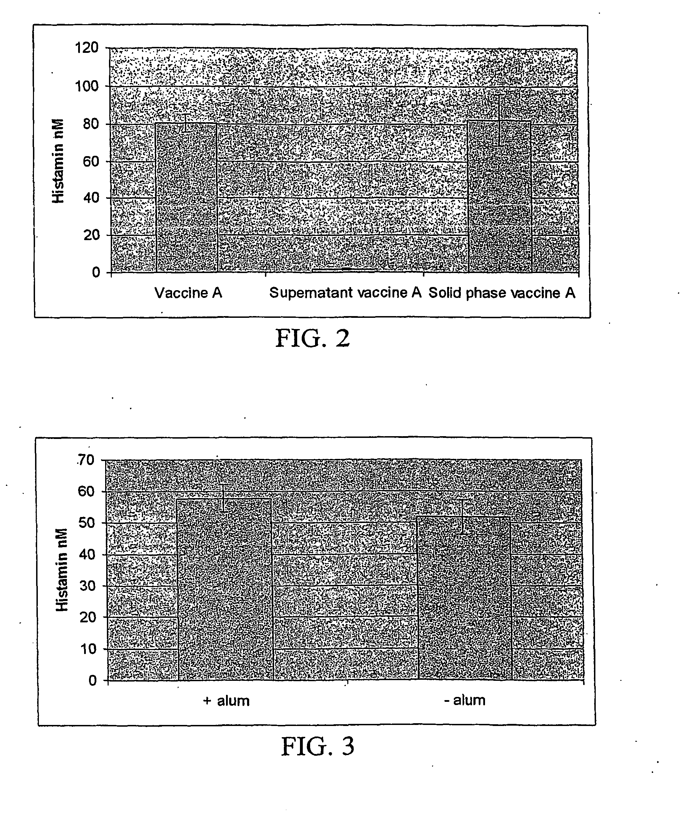

In a first experiment, a vaccine in the form of Phl p extract allergen adsorbed to aluminium hydroxide gel adjuvant (Vaccine A), the supernatant of Vaccine A after sedimentation, and the solid phase of Vaccine A after sedimentation were assayed using the histamine release assay described above. The supernatant and solid phase samples were obtained as follows: Vaccine A was allowed to stand for a period of three days in a vial to precipitate the gel phase, and a sample was taken with a syringe from the top of the vial, and from the bottom of the vial, respectively. The results are shown in Fig. 2.
As will appear from Fig. 2, only a very small amount of the allergen is present in the supernatant, and almost all of the allergen is present in the gel phase of the vaccine. This is indicative of an effective and safe depot vaccine.

In a second experiment, a vaccine in the form of Phl p extract allergen adsorbed to aluminium hydroxide gel adjuvant (+alum) and Phl p extract allergen in solution (-alum) were assayed using the histamine release assay described above. The results are shown in Fig. 3.

As will appear from Fig. 3, the histamine release elicited by the allergen gel vaccine is of the same level as that of the allergen solution, and hence the present histamine assay is capable of measuring the full allergenic activity of the gel vaccine.

## Example 3

### T cell proliferation assay for allergen adsorbed to an aluminium hydroxide gel adjuvant

T-cell assay:

Early events in the T-cell activation were investigated through flow cytometric analysis of T-cell expression of the surface receptor CD69, which are up-regulated at different time-points during T-cell activation.
Flow cytometric analysis is based on the attachment of fluorescence conjugated anti-surface marker antibodies to the cell surface and subsequent detection of the level fluorescence intensity on the individual cell by FACS analysis.

Phl p extract vaccines and supernatants of Phl p extract vaccines stored for 14 months, 8 months, 4 months and 1 month were tested. Also, two Phl p extracts (IMP 1 and IMP 4), purified Phl p 1 and purified Phl p 5 in solution were tested. Superantigen (SEB) and medium (med) were as references.

Results:

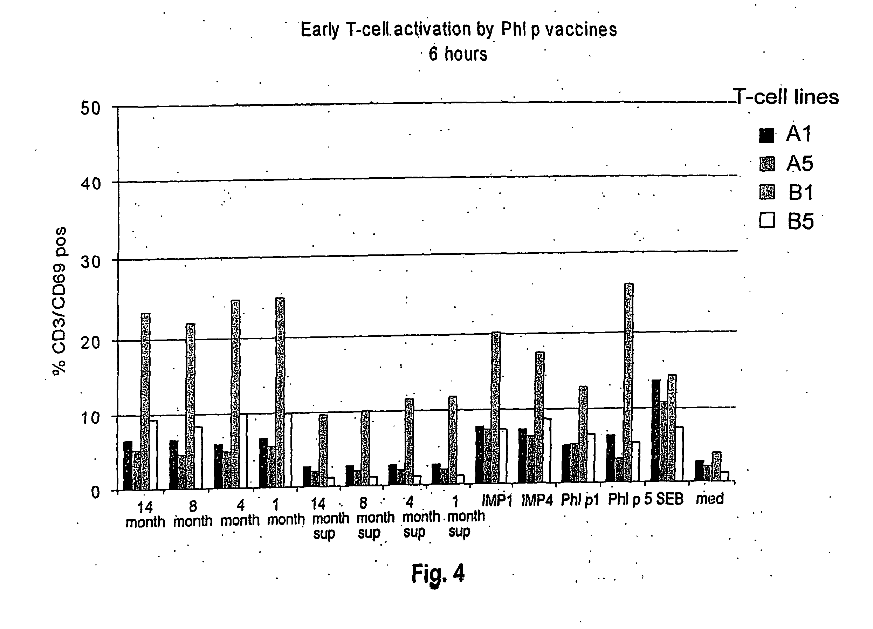

Four different T-cell lines where stimulated with complete grass vaccines or the supernatants of the vaccines obtained after centrifugation. The age of the vaccines differed from 1 to 14 month after end of production and the percentage of T-cells (CD3+) expressing CD69 was used as readout.
The results are shown in Fig. 4, which clearly demonstrates that vaccines of different age induce comparable T-cell activation and that it is possible to distinguish between the potential of the complete vaccine and the related supernatants.
In addition, the complete vaccines induce T-cell activation at the same level as the crude extracts (IMP1 and 4). The purified allergens Phl p 1, Phl p 5, and the super-antigen SEB are included as controls. In subsequent experiments it was shown that aluminium hydroxide gel without allergen does not induce CD69 expression on allergen-specific T-cells.

## Example 4

### Displacement (desorption) of allergen adsorbed to an aluminium hydroxide gel adjuvant

Materials:

Two aluminium hydroxide gel vaccines (Alutard) comprising the allergens Phl p 1 and Phl p 5 were tested. The displacement agents used were: 200 mM phosphate (NAH2PO4-Na2HPO4) buffer pH 7.4 diluted (in the sample vaccine) to either 5.0 mM or 50 mM phosphate buffer, serum pool constructed from samples from non allergic individuals (AG-525-) or serum pool containing 5 mM phosphate.

Methods:

The displacements were performed as follows: A vaccine (1 mL) was mixed either with an appropriate volume of 200 mM phosphate buffer yielding a final phosphate concentration of 5 or 50 mM and incubated 1 or 20 hours at 37°C; or diluted 1:1 with either serum pool alone or serum pool and an appropriate volume of 200 mM phosphate buffer yielding a final phosphate concentration of 5 mM. After incubation the samples were centrifuged (10 min, 4000 rpm) and the supernatant was harvested and stored at -20°C until used. Samples serving as reference or zero points were just centrifuged and the supernatant was stored at -20°C until used.
The amount of the individual allergens Phl p 1 and Phl p 5 were determined from inhibition experiments, in short: Standard curves for each allergen were obtained from inhibition experiments using biotinylated purified allergen inhibited with purified allergen. The data was fitted to a four parameter logistic function (Eqn. 1) and the inhibitory capacity measured for a given displacement supernatant was transformed into an allergen concentration using equation 1 and the parameters determined for each allergen. All the estimated concentrations were then corrected for dilutions with the displacement agents and all reported figures refer to the amount in 1 mL vaccine.

$$Y = B + \frac{T - B}{1 + 10^{(\log_{10} EC50 - \log_{10} X) \cdot HillSlope}} \qquad (1)$$

Results:

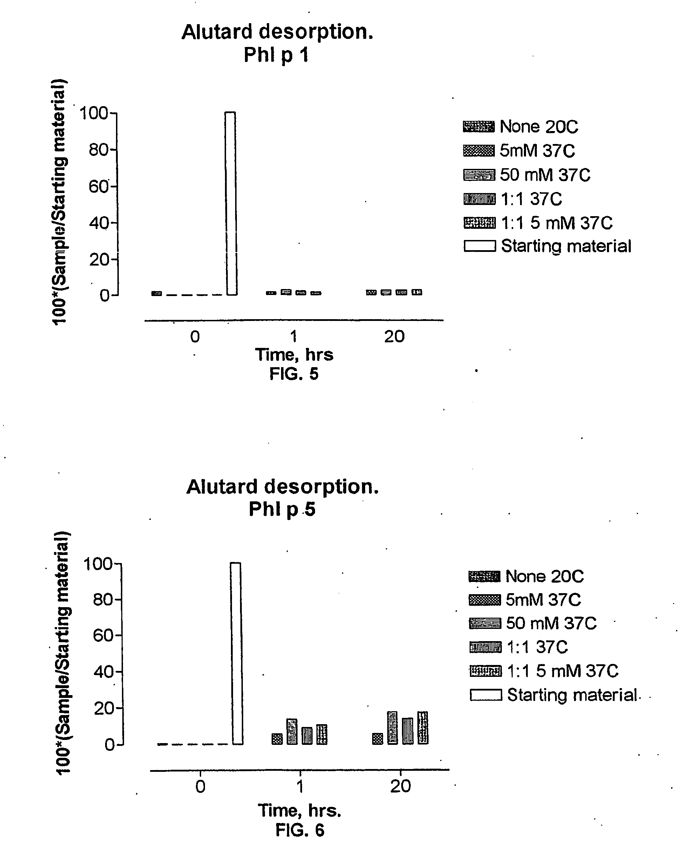

The results are shown in Figs. 5 and 6 using the following denominations: None: signifies no treatment of the sample, 1:1: signifies displacement performed with human serum and the molar figures refer to the final concentration of phosphate buffer. Also, the temperature is indicated. All figures represent the amount in the initial samples (dilution corrected).

As will appear from Figs. 5 and 6, the displacement of Phl p 1. from the aluminium hydroxide gel is much lower than the displacement of Phl p 5. However, in both cases the level of displacement is low. This is indicative of an effective and safe depot vaccine.

**Claims**

1. An in vitro method of evaluating the immunological activity of a vaccine preparation in the form of a mixture of a molecular protein antigen and a carrier in the form of aluminium hydroxide, wherein the mixture comprises a liquid phase and a solid phase, to which at least a part of the antigen is attached, the method comprising the steps of

   i) subjecting the vaccine to one or more measurements selected from the group consisting of:

      1) the immunological activity of the mixture,
      2) the immunological activity of antigen in the liquid phase,
      3) the immunological activity of antigen in the solid phase,
      4) the immunological activity of antigen in the liquid phase upon a treatment of the mixture to displace the antigen from the solid phase, and
      5) the immunological activity of antigen in the solid phase upon a treatment of the mixture to displace the antigen from the solid phase,

   wherein the immunological activity measurement is selected from the group consisting of a) antibody binding capacity using an immunoassay employing an antigen-specific antibody bound to an antibody solid phase, b) ability to activate effector cells and c) potential for inducing anaphylaxis; and
   ii) using the measurement results to evaluate the immunological activity of the vaccine.

2. A method according to claim 1, wherein the immunological activity is measured as the antibody binding capacity.

3. A method according to claim 2, wherein the antibody used or detected is selected from the group consisting of IgA, IgE, IgG, IgM and combinations thereof.

4. A method according to claim 3, wherein the antibodies used or detected are both IgE and IgG.

5. A method according to claim 3, wherein the antibody used or detected is IgE.

6. A method according to any of claims 2-5, wherein the immunological activity is measured in an immunoassay.

7. A method according to claim 6, wherein the immunoassay is a competitive immunoassay.

8. A method according to claim 1, wherein the immunological activity is measured as the ability to activate effector cells of the immune system.

9. A method according to claim 8, wherein whole blood is used for effector cells activation.

10. A method according to claim 8, wherein the effector cells are cells isolated from a biological sample.

**11.** A method according to claim 8, wherein the effector cells are cells isolated from a biological sample and cultivated.

**12.** A method according to claim 8, wherein the effector cells are cells isolated from a biological sample, cultivated and modified.

**13.** A method according to any of claims 10-12, wherein the effector cells are selected from the group consisting of mast cells, basophils, eosinophils, T cells, B cells and Antigen Presenting Cells (APC), and combinations thereof.

**14.** A method according to any of claims 8-13, wherein the effector cell activating ability is measured by measuring the level of an effector cell marker.

**15.** A method according to claim 14, wherein the marker is selected from the group consisting of secretory molecules, surface molecules and intracellular molecules.

**16.** A method according to claim 15, wherein the secretory molecule is selected from the group consisting of mediators, cytokines, cytotoxic proteins and soluble receptors.

**17.** A method according to claim 16, wherein the mediator measured is selected from the group consisting of histamine, leucotrienes ($LTB_4$, $LTC_4$, $LTD_4$ and $LTE_4$), prostaglandines ($PGD_2$, $PGE_2$ and $PGF_{2a}$), thromboxane, Platelet Activating Factor (PAF), Major Basic Protein (MBP), Eosinophil Cationic Protein (ECP), Eosinophil Derived Neurotoxin (EDN), Eosinophil Peroxidase (EPO), bradykinin, adenosine, Substance P, Neurokinin A, complement factors (e.g. C3d), including complement fragments; Serotonin, Oxygen Radicals, basogranulin, and mast cell and basophil proteases, including tryptase, chymase, carboxypeptidase and cathepsin.

**18.** A method according to claim 16, wherein the cytokine measured is selected from the group consisting of Interleukins (IL-1 to IL-27), hematopoietric growth factors, granulocyte-macrophage colony stimulating factors (e.g. CM-CSF), interferons (IFN$\alpha$, IFN$\beta$, IFN$\gamma$), TNF, lymphotoxin, immunoglobulin superfamily members (Intercellular Adhesion Molecule-1 (ICAM-1), Vascular Cell Adhesion Molecule-1 (VCAM-1)), the TGF-beta family and the chemokines (IL-8, RANTES).

**19.** A method according to claim 16, wherein the cytotoxic protein measured is selected from the group consisting of Eosinophil Cationic Protein (ECP), Major Basic Protein (MBP) and Eosinophil Derived Neurotoxin (EDN).

**20.** A method according to claim 15, wherein the surface molecule is selected from the group consisting of surface receptors and adhesion molecules.

**21.** A method according to claim 20, wherein the surface moiecuie is selected from the group consisting of selectins, integrins, immunoglobulin superfamily members (Intercellular Adhesion Molecule-1 (ICAM-1), Vascular Cell Adhesion Molecule-1 (VCAM-1)), Very Late Activation Antigen-4 (VLA4), CD11B, CD11C, CD18, CD23, CD69, CD203C, CD31, CD162, CD162L and basogranulin.

**22.** A method according to any of claims 8-13, wherein the effector cell activating ability is measured by measuring the T cell proliferation.

**23.** A method according to claim 1, wherein the immunological activity is the potential for inducing anaphylaxis.

**24.** A method according to claim 23, wherein the potential for inducing anaphylaxis is measured by measuring the level of an effector cell anaphylaxis marker selected from the group consisting of histamine, tryptase, basogranulin, leucotrien LTC4, CD63, CD69 and CD203C.

**25.** A method according to claim 23 or 24, wherein the potential for inducing anaphylaxis in whole blood is measured.

**26.** A method according to claim 25, wherein the whole blood used for the measurement has been withdrawn from a subject maximally five hours prior to the measurement.

**27.** A method according to claim 25, wherein the whole blood used for the measurement has been withdrawn from a subject maximally two hours prior to the measurement.

28. A method according to any of claims 25-27, wherein the vaccine and the whose blood is mixed in a ratio corresponding to a calculated in vivo ratio, which would occur in a subject as a result of an accidental administration of a vaccine dose into the blood stream of a subject.

29. A method according to any of claims 25-28, wherein whole blood adjusted to body temperature is used.

30. A method according to claim 23 or 24 comprising measuring the potential for inducing anaphylaxis in effector cells isolated from a biological sample.

31. A method according to claim 23 or 24 comprising measuring the potential for inducing anaphylaxis in 1) in effector cells isolated from a biological sample, 2) in effector cells isolated from a biological sample and cultivated, or 3) in effector cells isolated from a biological sample, cultivated and modified.

32. A method according to claim 31 comprising measuring the potential for inducing anaphylaxis in effector cells isolated from a biological sample, cultivated and modified.

33. A method according to claim 32, wherein the modified effector cells are genetically modified cells or malignantly transformed cells.

34. A method according to any of claims 29 or 30, wherein the effector cells are selected from the group consisting of mast cells, basophils, eosinophils, T cells, B cells and Antigen Presenting Cells (APC), and combinations thereof.

35. A method according to claim 1, wherein the vaccine is subjected solely to a measurement of the immunological activity of the mixture of the liquid phase and the solid phase (measurement 1)).

36. A method according to claim 1, wherein the vaccine is subjected soieiy to a measurement of the immunological activity of antigen in the liquid phase upon a treatment of the mixture to displace the antigen from the solid phase (measurement 4)).

37. A method according to claim 1, the vaccine is subjected both to a measurement of the immunological activity of the mixture of the liquid phase and the solid phase (measurement 1)), and to a measurement of the immunological activity of antigen in the liquid phase (measurement 2)).

38. A method according to claim 1, wherein the vaccine is subjected both to a measurement of the immunological activity of antigen in the liquid phase (measurement 2)), and to a measurement of the immunological activity of antigen in the solid phase (measurement 3)).

39. A method according to claim 1, wherein the displacing treatment comprises contacting the mixture with a protein-containing reagent or an anion.

40. A method according to claim 39, wherein the protein-containing reagent is a body fluid containing proteins.

41. A method according to claim 40, wherein the body fluid is blood serum.

42. A method according to claim 39, wherein anion is phosphate ions.

43. A method according to any of the preceding claims, wherein the immunological activity of an antigen-containing intermediate product used for preparing the vaccine is measured, and wherein the evaluation of the immunological activity of the vaccine is based on a comparison of the measurement result obtained for the intermediate product and the measurement results obtained for one or more of measurements 1) - 5).

44. A method according to any of the preceding claims, wherein the vaccine is subjected to the measurements immediately upon preparation.

45. A method according to claim 44, wherein the vaccine is subjected to the measurements immediately after preparation and after one or more periods of storage, and wherein the evaluation of the immunological activity of the vaccine is based on a comparison between the former and latter measurement results.

46. A method according to any of the preceding claims, wherein the evaluation of the immunological activity of the vaccine is based on a comparison between the measurement results obtained for the vaccine and prior measurement results from the same type of vaccine or from another type of vaccine, wherein the same type of immunological activity measurement is used to obtain the measurement results for the vaccine and the prior measurement results.

47. A method according to any of the preceding claims, wherein the molecular protein antigen is an allergen.

48. A method according to claim 47, wherein the allergen is selected from the group consisting of tree pollen allergens, grass pollen allergens, herb pollen allergens, mite allergens, venom allergens, animal hair and dandruff allergens and food allergens.

49. A method according to claim 48, wherein the allergen is a grass pollen allergen.

50. A method according to claim 48, wherein the allergen is a dust mite allergen.

51. A method according to any of claims 47-50, wherein the allergen is a mixture of at least two different allergen species.

52. A method according to any of claims 47-51, wherein the allergen is in the form of an extract, a purified allergen, a modified allergen or a recombinant allergen or a mutant of a recombinant allergen or any combination thereof.

53. A method according to any of claims 47-52, wherein the allergen is in the form of an extract.

54. A method according to claim 53, wherein the immunological activity of two or more major and/or minor allergens of the extract are measured.

55. A method according to claim 54, wherein in addition the immunological activity of the whole extract is measured.

56. A method of preparing a vaccine preparation in the form of a mixture of a molecular protein antigen and a solid phase carrier, in the form of aluminium hydroxide, wherein the mixture comprises a liquid phase and a solid phase, to which at least a part of the antigen is attached, the method comprising

i) mixing the antigen and the carrier,
ii) measuring the immunological activity of the vaccine using the method according to any of claims 1-54, and
iii) optionally repeating steps i) and ii) until a desired immunological activity is obtained.

**Patentansprüche**

1. In-vitro-Verfahren zum Evaluieren der immunologischen Aktivität einer Impfstoff-Zusammensetzung in Form einer Mischung aus einem molekularen Protein-Antigen und einem Träger in Form von Aluminiumhydroxid, wobei die Mischung eine Flüssigphase und eine Festphase aufweist, an welche mindestens ein Teil des Antigens angeheftet ist; wobei das Verfahren die Schritte aufweist:

i) Unterwerfen des Impfstoffes einer oder mehrerer aus der Gruppe ausgewählten Messungen, bestehend aus:

1) der immunologischen Aktivität der Mischung,
2) der immunologischen Aktivität des Antigens in der Flüssigphase,
3) der immunologischen Aktivität des Antigens in der Festphase,
4) der immunologischen Aktivität des Antigens in der Flüssigphase nach einer Behandlung der Mischung zur Verdrängung des Antigens von der Festphase, und
5) der immunologischen Aktivität des Antigens in der Festphase nach einer Behandlung der Mischung zur Verdrängung des Antigens von der Festphase, wobei die immunologische Aktivitätsmessung aus der Gruppe ausgewählt wird, bestehend aus a) einer Antikörper-Bindefähigkeit, welche einen Immuntest unter Einsetzen eines an eine Antikörper-Festphase gebundenen Antigen-spezifischen Antikörpers verwendet, b) einer Fähigkeit zum Aktivieren von Effektorzellen und c) einem Potenzial zum Induzieren einer Anaphylaxie; und

ii) Verwenden der Messergebnisse zum Evaluieren der immunologischen Aktivität des Impfstoffes.

2. Verfahren nach Anspruch 1, wobei die immunologische Aktivität als Antikörper-Bindefähigkeit gemessen wird.

3. Verfahren nach Anspruch 2, wobei der verwendete oder aufgefundene Antikörper aus der Gruppe, bestehend aus IgA, IgE, IgG, IgM und Kombinationen davon, ausgewählt wird.

4. Verfahren nach Anspruch 3, wobei die verwendeten oder aufgefundenen Antikörper sowohl IgE als auch IgG sind.

5. Verfahren nach Anspruch 3, wobei der verwendete oder aufgefundenen Antikörper IgE ist.

6. Verfahren nach einem der Ansprüche 2-5, wobei die immunologische Aktivität in einem Immuntest gemessen wird.

7. Verfahren nach Anspruch 6, wobei der Immuntest ein kompetitiver Immuntest ist.

8. Verfahren nach Anspruch 1, wobei die immunologische Aktivität als Fähigkeit zum Aktivieren von Effektorzellen des Immunsystems gemessen wird.

9. Verfahren nach Anspruch 8, wobei Vollblut zur Effektorzellen-Aktivierung verwendet wird.

10. Verfahren nach Anspruch 8, wobei die Effektorzellen aus einer biologischen Probe isolierte Zellen sind.

11. Verfahren nach Anspruch 8, wobei die Effektorzellen aus einer biologischen Probe isolierte und gezüchtete Zellen sind.

12. Verfahren nach Anspruch 8, wobei die Effektorzellen aus einer biologischen Probe isolierte, gezüchtete und modifizierte Zellen sind.

13. Verfahren nach einem der Ansprüche 10-12, wobei die Effektorzellsn aus der Gruppe, bestehend aus Mastzellen, Basophilen, Eosinophilen, T-Zellen, B-Zellen und Antigen-präsentierenden Zellen (Antigen Presenting Cells (APC)), und Kombinationen davon, ausgewählt werden.

14. Verfahren nach einem der Ansprüche 8-13, wobei die Effektorzellen-Aktivierungsfähigkeit durch Messen des Pegels eines Effektorzellen-Markers gemessen wird.

15. Verfahren nach Anspruch 14, wobei der Marker aus der Gruppe, bestehend aus sekretorischen Molekülen, Oberflächenmolekizlen und Intrazellularmolekülen, ausgewählt wird.

16. Verfahren nach Anspruch 15, wobei das sekretorische Molekül aus der Gruppe, bestehend aus Mediatoren, Zytokinen, zytotoxischen Proteinen und lösbaren Rezeptoren, ausgewählt wird.

17. Verfahren nach Anspruch 16, wobei der gemessene Mediator aus der Gruppe, bestehend aus Histamin, Leukotrienen ($LTB_4$, $LTC_4$, $LTD_4$, und $LTE_4$), Prostaglandinen ($PGD_2$, $PGE_2$, $PGF_{2a}$), Thromboxan, Plättchen-aktivierendem Faktor (Platelet Activating Factor (PAF)), höherem Basisprotein (Major Basic Protein (MBP)), eosinophilem kationischen Protein (Eosinophil Cationic Protein (ECP)), eosinophilem abgeleiteten Neurotoxin (Eosinophil Derived Neurotoxin (EDN)), Eosinophil Peroxidase (EPO), Bradykinin, Adenosin, Substanz P, Neurokinin A, Komplementfaktoren (z.B. C3d), einschließlich Komplementfragmenten; Serotonin, Sauerstoffradikalen, basophilem Granulin, und Mastzellen und basophilen Proteasen, einschließlich Tryptase, Chymase, Carboxypeptidase und Kathepsin, ausgewählt wird.

18. Verfahren nach Anspruch 16, wobei das gemessene Zytokin aus der Gruppe, bestehend aus Interleukinen (IL-1 bis IL-27), Blut-bildenden Wachstumsfaktoren, Granulozyt-Makrophagen-Kolonie-stimulierenden Faktoren (z.B. CM-CSF), Interferonen (IFN$\alpha$, IFN$\beta$, IFN$\gamma$), TNF, Lymphotoxin, Immunglobulin-Überfamilien-Mitgliedern (Interzellularem Adhäsions-Molekül-1 (Intercellular Adhesion Molecule-1 (ICAM-1)); vaskulärem Zelladhäsions-Molekül-1 (Vascular Cell Adhesion Molecule-1 (VCAM-1))), der TGF-$\beta$-Familie und den Chemokinen (IL-8, RANTES), ausgewählt wird.

19. Verfahren nach Anspruch 16, wobei das gemessene zytotoxische Protein aus der Gruppe, bestehend aus eosinophilem kationischen Protein (Eosinophil Cationic Protein (ECP)), Major Basic Protein (MBP) und eosinophilem abgeleiteten Neurotoxin (Eosinophil Derived Neurotoxin (EDN)), ausgewählt wird.

**20.** Verfahren nach Anspruch 15, wobei das Oberflächenmolekül aus der Gruppe, bestehend aus Oberflächen-Rezeptoren und Adhäsions-Molekülen, ausgewählt wird.

**21.** Verfahren nach Anspruch 20, wobei das Oberflächenmolekül aus der Gruppe, bestehend aus Selektinen, Integrinen, Immunglobulin-Überfamilicn-Mitgliedern (interzellularem Adhäsions-Molekül-1 (Intercellular Adhesion Molecule-1 (ICAM-1)), vaskulärem Zell-Adhäsions-Molekül-1 (Vascular Cell Adhesion Molecule-1 (VCAM-1))), sehr spätem Aktivierungs-Antigen-4 (Very-Late-Activation-Antigen-4 (LVA4)), CD11B, CD11C, CD18, CD23, CD69, CD203C, CD31, CD162, CD162L und basophilem Granulin, ausgewählt wird.

**22.** Verfahren nach einem der Ansprüche 8-13, wobei die Effektorzellen-Aktivierungsfähigkeit durch Messen der T-Zellen-Proliferation gemessen wird.

**23.** Verfahren nach Anspruch 1, wobei die immunologische Aktivität das Potenzial zum Induzieren von Anaphylaxie ist.

**24.** Verfahren nach Anspruch 23, wobei das Potenzial zum Induzieren von Anaphylaxie durch Messen des Pegels eines Effektorzellen-Anaphylaxie-Markers, ausgewählt aus der Gruppe, bestehend aus Histamin, Tryptase, basophilem Granulin, Leukotrien LTC4, CD63, CD69 und CD203C, gemessen wird.

**25.** Verfahren nach Anspruch 23 oder 24, wobei das Potenzial zum Induzieren von Anaphylaxie in Vollblut gemessen wird.

**26.** Verfahren nach Anspruch 25, wobei das für die Messung verwendete Vollblut einer Testperson maximal fünf Stunden vor der Messung abgenommen worden ist.

**27.** Verfahren nach Anspruch 25, wobei das für die Messung verwendete Vollblut einer Testperson maximal zwei Stunden vor der Messung abgenommen worden ist.

**28.** Verfahren nach einem der Ansprüche 25-27, wobei der Impfstoff und das Vollblut in einem Verhältnis entsprechend einem berechneten In-vivo-Verhältnis gemischt wird, welches in einer Testperson als Ergebnis einer zufälligen Zufuhr einer Impfstoff Dosis in den Blutstrom einer Testperson entstehen könnte.

**29.** Verfahren nach einem der Ansprüche 25-28, wobei an Körpertemperatur angepasstes Vollblut verwendet wird.

**30.** Verfahren nach Anspruch 23 oder 24, welches das Messen des Potenzials zum Induzieren von Anaphylaxie in von einer biologischen Probe isolierten Effektorzellen aufweist.

**31.** Verfahren nach Anspruch 23 oder 24, welches das Messen des Potenzials zum Induzieren von Anaphylaxie in 1) in von einer biologischen Probe isolierten Effektorzellen, 2) in von einer biologischen Probe isolierten und gezüchteten Effektorzellen, oder 3) in von einer biologischen Probe isolierten, gezüchteten und modifizierten Effektorzellen aufweist.

**32.** Verfahren nach Anspruch 31, welches das Messen des Potenzials zum Induzieren von Anaphylaxie in von einer biologischen Probe isolierten, gezüchteten und modifizierten Effektorzellen aufweist.

**33.** Verfahren nach Anspruch 32, wobei die modifizierten Effektorzellen genetisch modifizierte Zellen oder maligne transformierte Zellen sind.

**34.** Verfahren nach einem der Ansprüche 29 oder 30, wobei die EfFektorzellen aus der Gruppe, bestehend aus Mastzellen, Basophilen, Eosinophilen, T-Zellen, B-Zellen und Antigen-präsentierenden Zellen (Antigen Presenting Cells (APC)), und Kombinationen davon, ausgewählt werden.

**35.** Verfahren nach Anspruch 1, wobei der Impfstoff ausschließlich einer Messung der immunologischen Aktivität der Mischung der Flüssigphase und der Festphase (Messung 1)) unterworfen wird.

**36.** Verfahren nach Anspruch 1, wobei der Impfstoff ausschließlich einer Messung der immunologischen Aktivität eines Antigens in der Flüssigphase nach der Behandlung der Mischung, um das Antigen von der Festphase zu verdrängen (Messung 4)), unterworfen wird.

**37.** Verfahren nach Anspruch 1, wobei der Impfstoff sowohl einer Messung der immunologischen Aktivität der Mischung der Flüssigphase und der Festphase (Messung 1)), als auch einer Messung der immunologischen Aktivität eines Antigens in der Flüssigphase (Messung 2)) unterworfen wird.

**38.** Verfahren nach Anspruch 1, wobei der Impfstoff sowohl einer Messung der immunologischen Aktivität eines Antigens in der Flüssigphase (Messung 2)), als auch einer Messung der immunologischen Aktivität eines Antigens in der Festphase (Messung 3)) unterworfen wird.

**39.** Verfahren nach Anspruch 1, wobei die Verdrängungsbehandlung ein In-Kontakt-Bringen der Mischung mit einem Protein-enthaltenden Reagenz oder einem Anion beinhaltet.

**40.** Verfahren nach Anspruch 39, wobei das Protein-enthaltende Reagenz eine Körperflüssig ist, welche Proteine enthält.

**41.** Verfahren nach Anspruch 40, wobei die Körperflüssigkeit ein Blutserum ist.

**42.** Verfahren nach Anspruch 39, wobei das Anion Phosphationen ist.

**43.** Verfahren nach einem der vorhergehenden Ansprüche, wobei die immunologische Aktivität eines zur Herstellung des Impfstoffes verwendeten Antigen-enthaltenden Zwischenprodukts gemessen wird, und wobei die Evaluierung der immunologischen Aktivität des Impfstoffes auf einem Vergleich des für das Zwischenprodukt erhaltenen Messergebnisses mit den für eine oder mehrere Messungen 1)-5) erhaltenen Messergebnissen basiert.

**44.** Verfahren nach einem der vorhergehenden Ansprüche, wobei der Impfstoff den Messungen unmittelbar nach Herstellung unterworfen wird.

**45.** Verfahren nach Anspruch 44, wobei der Impfstoff den Messungen unmittelbar nach Herstellung und nach einer oder mehreren Lagerungsphasen unterworfen wird, und wobei die Evaluierung der immunologischen Aktivität des Impfstoffes auf einem Vergleich zwischen den ersteren und den letzteren Messergebnissen basiert.

**46.** Verfahren nach einem der vorhergehenden Ansprüche, wobei die Evaluierung der immunologischen Aktivität des Impfstoffes auf einem Vergleich zwischen den für den Impfstoff erhaltenen Messergebnissen und Ergebnissen vor Messung, ausgehend vom selben oder einem anderen Impfstoff-Typ, basiert, wobei die selbe Art der immunologischen Aktivitätsmessung zum Erhalten der Messergebnisse für den Impfstoff und der Ergebnisse vor Messung verwendet wird.

**47.** Verfahren nach einem der vorhergehenden Ansprüche, wobei das Molekularprotein-Antigen ein Allergen ist.

**48.** Verfahren nach Anspruch 47, wobei das Allergen aus der Gruppe, bestehend aus Baumpollen-Allergenen, Graspollen-Allergenen, Kräuterpollen-Allergenen, Milben-Allergenen, Tiergift-Allergenen, Tierhaar- und Tierschuppen-Allergenen, und Lebensmittel-Allergenen, ausgewählt wird.

**49.** Verfahren nach Anspruch 48, wobei das Allergen ein Graspollen-Allergen ist.

**50.** Verfahren nach Anspruch 48, wobei das Allergen ein Staubmilben-Allergen ist.

**51.** Verfahren nach einem der Ansprüche 47-50, wobei das Allergen eine Mischung aus mindestens zwei verschiedenen Allergen-Spezies ist.

**52.** Verfahren nach einem der Ansprüche 47-51, wobei das Allergen in Form eines Extrakts, eines gereinigten Allergens, eines modifizierten oder rekombinanten Allergens oder Mutanten eines rekombinanten Allergens oder irgendeiner Kombination davon ist.

**53.** Verfahren nach einem der Ansprüche 47-52, wobei das Allergen in Form eines Extrakts ist.

**54.** Verfahren nach Anspruch 53, wobei die immunologische Aktivität von zwei oder mehreren Haupt- und/oder Neben-Allergenen des Extrakts gemessen wird.

**55.** Verfahren nach Anspruch 54, wobei zusätzlich die immunologische Aktivität des ganzen Extrakts gemessen wird.

**56.** Verfahren zur Herstellung einer Impfstoff-Zusammensetzung in Form einer Mischung aus einem Molekularprotein-Antigen und einem Festphasen-Träger in Form von Aluminiumhydroxid, wobei die Mischung eine Flüssigphase und eine Festphase aufweist, an welche mindestens ein Teil des Antigens angeheftet ist; wobei das Verfahren aufweist:

  i) Mischen des Antigens und des Trägers,
  ii) Messen der immunologischen Aktivität des Impfstoffes unter Verwendung des Verfahrens nach einem der Ansprüche 1-54, und
  iii) gegebenenfalls Wiederholen der Schritte i) und ii), bis eine gewünschte immunologische Aktivität erreicht wird.

## Revendications

**1.** Procédé in vitro d'évaluation de l'activité immunologique d'une préparation de vaccin sous la forme d'un mélange d'un antigène de protéine moléculaire et d'un support sous la forme d'hydroxyde d'aluminium, dans lequel le mélange comprend une phase liquide et une phase solide, auquel au moins une partie de l'antigène est attachée, le procédé comprenant les étapes de :

  i) soumission du vaccin à une ou plusieurs mesures choisies parmi le groupe constitué de :

    1) l'activité immunologique du mélange,
    2) l'activité immunologique de l'antigène dans la phase liquide,
    3) l'activité immunologique de l'antigène dans la phase solide,
    4) l'activité immunologique de l'antigène dans la phase liquide lors d'un traitement du mélange pour déplacer l'antigène de la phase solide, et
    5) l'activité immunologique de l'antigène dans la phase solide lors d'un traitement du mélange pour déplacer l'antigène de la phase solide,

  dans lequel la mesure de l'activité immunologique est choisie parmi le groupe constitué de a) la capacité de liaison à l'anticorps en utilisant un dosage immunologique utilisant un anticorps spécifique de l'antigène lié à une phase solide de l'anticorps, b) la capacité d'activation des cellules effectrices et c) le potentiel d'induction de l'anaphylaxie ; et
  ii) l'utilisation des résultats des mesures pour évaluer l'activité immunologique du vaccin.

**2.** Procédé selon la revendication 1, dans lequel l'activité immunologique est mesurée comme la capacité de liaison de l'anticorps.

**3.** Procédé selon la revendication 2, dans lequel l'anticorps utilisé ou détecté est choisi parmi le groupe constitué d'IgA, IgE, IgG, IgM et des combinaisons de celles-ci.

**4.** Procédé selon la revendication 3, dans lequel les anticorps utilisés ou détectés sont constitués à la fois d'IgE et d'IgG.

**5.** Procédé selon la revendication 3, dans lequel l'anticorps utilisé ou détecté est constitué d'IgE.

**6.** Procédé selon l'une quelconque des revendications 2-5, dans lequel l'activité immunologique est mesurée dans un dosage immunologique.

**7.** Procédé selon la revendication 6, dans lequel le dosage immunologique est un dosage immunologique de type compétitif.

**8.** Procédé selon la revendication 1, dans lequel l'activité immunologique est mesurée comme la capacité d'activation des cellules effectrices du système immunitaire.

**9.** Procédé selon la revendication 8, dans lequel le sang entier est utilisé pour l'activation des cellules effectrices.

**10.** Procédé selon la revendication 8, dans lequel les cellules effectrices sont des cellules isolées d'un échantillon biologique.

**11.** Procédé selon la revendication 8, dans lequel les cellules effectrices sont des cellules isolées d'un échantillon biologique et cultivées.

**12.** Procédé selon la revendication 8, dans lequel les cellules effectrices sont des cellules isolées d'un échantillon biologique, cultivées et modifiées.

**13.** Procédé selon l'une quelconque des revendications 10-12, dans lequel les cellules effectrices sont choisies parmi le groupe constitué de mastocytes, de basophiles, d'éosinophiles, de cellules T, de cellules B et de cellules présentant l'antigène (APC) et des combinaisons de ceux-ci.

**14.** Procédé selon l'une quelconque des revendications 8-13, dans lequel la capacité d'activation des cellules effectrices est mesurée en mesurant le taux d'un marqueur des cellules effectrices.

**15.** Procédé selon la revendication 14, dans lequel le marqueur est choisi parmi le groupe constitué de molécules sécrétoires, de molécules de surface et de moléculaires intracellulaires.

**16.** Procédé selon la revendication 15, dans lequel la molécule sécrétoitre est choisie parmi le groupe constitué des médiateurs, des cytokines, des protéines cytotoxiques et des récepteurs solubles.

**17.** Procédé selon la revendication 16, dans lequel le médiateur mesuré est choisi parmi le groupe constitué de l'histamine, des leucotriènes ($LTB_4$, $LTC_4$, $LTD_4$ et $LTE_4$), des prostaglandines ($PGD_2$, $PGE_2$ et $PGF_{2a}$), du thromboxane, du facteur d'activation des plaquettes (PAF), de la protéine basique majeure (MBP), de la protéine cationique éosinophile (ECP), de la neurotoxine dérivée d'éosinophile (EDN), de la peroxydase d'éosinophile (EPO), de la bradykinine, de l'adénosine, de la substance P, de la neurokinine A, des facteurs du complément (par exemple, C3d), incluant des fragments du complément; de la sérotonine, des radicaux d'oxygène, de la basogranuline, et des protéases des mastocytes et des basophiles, incluant la tryptase, la chymase, la carboxypeptidase et la cathepsine.

**18.** Procédé selon la revendication 16, dans lequel la cytokine mesurée est choisie parmi le groupe constitué des interleukines (IL-1 à IL-27), des facteurs de croissance hématopoïétiques, des facteurs stimulant les colonies de granulocyte-macrophage (par exemple, CM-CSF), des interférons ($IFN\alpha$, $IFN\beta$, $IFN\gamma$), du TNF, de la lymphotoxine, des membres de la superfamille des immunoglobulines (molécule-1 d'adhésion intercellulaire (ICAM-1), la molécule-1 d'adhésion cellulaire vasculaire (VCAM-1)), la famille de TGF-beta et les chimiokines (IL-8, RANTES).

**19.** Procédé selon la revendication 16, dans lequel la protéine cytotoxique mesurée est choisie parmi le groupe constitué de la protéine cationique éosinophile (ECP), la protéine basique majeure (MBP) et la neurotoxine dérivée d'éosinophile (EDN).

**20.** Procédé selon la revendication 15, dans lequel la molécule de surface est choisie parmi le groupe constitué des récepteurs de surface et des molécules d'adhésion.

**21.** Procédé selon la revendication 20, dans lequel la molécule de surface est choisie parmi le groupe constitué des sélectines, des intégrines, des membres de la superfamille des immunoglobulines (molécule-1 d'adhésion intercellulaire (ICAM-1), la molécule-1 d'adhésion cellulaire vasculaire (VCAM-1)), l'antigène-4 d'activation très tardive (VLA4), CD11B, CD11C, CD18, CD23, CD69, CD203C, CD31, CD162, CD162L et la basogranuline.

**22.** Procédé selon l'une quelconque des revendications 8-13, dans lequel la capacité d'activation des cellules effectrices est mesurée en mesurant la prolifération des cellules T.

**23.** Procédé selon la revendication 1, dans lequel l'activité immunologique est le potentiel d'induction de l'anaphylaxie.

**24.** Procédé selon la revendication 23, dans lequel le potentiel d'induction de l'anaphylaxie est mesuré en mesurant le taux d'un marqueur d'anaphylaxie des cellules effectrices choisi parmi le groupe constitué de l'histamine, la tryptase, la basogranuline, le leucotriène LTC4, CD63, CD69 et CD203C.

**25.** Procédé selon les revendications 23 ou 24, dans lequel le potentiel d'induction de l'anaphylaxie dans le sang entier est mesuré.

**26.** Procédé selon la revendication 25, dans lequel le sang entier utilisé pour la mesure a été prélevé chez un sujet cinq heures au plus avant la mesure.

27. Procédé selon la revendication 25, dans lequel le sang entier utilisé pour la mesure a été prélevé chez un sujet deux heures au plus avant la mesure.

28. Procédé selon l'une quelconque des revendications 25-27, dans lequel le vaccin et le sang entier sont mélangés dans un rapport correspondant à un rapport in vivo calculé, qui apparaîtrait chez un sujet comme le résultat d'une administration accidentelle d'une dose de vaccin dans le circuit sanguin d'un sujet.

29. Procédé selon l'une quelconque des revendications 25-28, dans lequel on utilise du sang entier ajusté à la température corporelle.

30. Procédé selon les revendications 23 ou 24 comprenant la mesure du potentiel d'induction de l'anaphylaxie dans les cellules effectrices isolées d'un échantillon biologique.

31. Procédé selon les revendications 23 ou 24 comprenant la mesure du potentiel d'induction de l'anaphylaxie 1) dans des cellules effectrices isolées d'un échantillon biologique, 2) dans des cellules effectrices isolées d'un échantillon biologique et cultivées, ou 3) dans des cellules effectrices isolées d'un échantillon biologique, cultivées et modifiées.

32. Procédé selon la revendication 31 comprenant la mesure du potentiel d'induction de l'anaphylaxie dans des cellules effectrices isolées d'un échantillon biologique, cultivées et modifiées.

33. Procédé selon la revendication 32, dans lequel les cellules effectrices modifiées sont des cellules génétiquement modifiées ou des cellules transformées de manière maligne.

34. Procédé selon l'une quelconque des revendications 29 ou 30, dans lequel les cellules effectrices sont choisies parmi le groupe constitué des mastocytes, des basophiles, des éosinophiles, des cellules T, des cellules B et des cellules présentant l'antigène (APC), et des combinaisons de ceux-ci.

35. Procédé selon la revendication 1, dans lequel le vaccin est soumis uniquement à une mesure de l'activité immunologique du mélange de la phase liquide et de la phase solide (mesure 1)).

36. Procédé selon la revendication 1, dans lequel le vaccin est soumis uniquement à une mesure de l'activité immunologique de l'antigène dans la phase liquide lors d'un traitement du mélange pour déplacer l'antigène de la phase solide (mesure 4)).

37. Procédé selon la revendication 1, dans lequel le vaccin est soumis à la fois à une mesure de l'activité immunologique du mélange de la phase liquide et de la phase solide (mesure 1)), et à une mesure de l'activité immunologique de l'antigène dans la phase liquide (mesure 2)).

38. Procédé selon la revendication 1, dans lequel le vaccine est soumis à la fois à une mesure de l'activité immunologique de l'antigène dans la phase liquide (mesure 2)), et à une mesure de l'activité immunologique de l'antigène dans la phase solide (mesure 3)).

39. Procédé selon la revendication 1, dans lequel le traitement de déplacement comprend la mise en contact du mélange avec un réactif contenant une protéine ou un anion.

40. Procédé selon la revendication 39, dans lequel le réactif contenant une protéine est un fluide corporel contenant des protéines.

41. Procédé selon la revendication 40, dans lequel le fluide corporel est du sérum sanguin.

42. Procédé selon la revendication 39, dans lequel l'anion est constitué d'ions phosphate.

43. Procédé selon l'une quelconque des revendications précédentes, dans lequel l'activité immunologique d'un produit intermédiaire contenant l'antigène utilisé pour préparer le vaccin est mesurée, et dans lequel l'évaluation de l'activité immunologique du vaccin est basée sur une comparaison du résultat de la mesure obtenu pour le produit intermédiaire et les résultats des mesures obtenus pour une ou plusieurs des mesures 1)-5).

**44.** Procédé selon l'une quelconque des revendications précédentes, dans lequel le vaccin est soumis aux mesures immédiatement après la préparation.

**45.** Procédé selon la revendication 44, dans lequel le vaccin est soumis aux mesures immédiatement après préparation et après une ou plusieurs périodes de stockage, et dans lequel l'évaluation de l'activité immunologique du vaccin est basée sur une comparaison entre les résultats des premières et dernières mesures.

**46.** Procédé selon l'une quelconque des revendications précédentes, dans lequel l'évaluation de l'activité immunologique du vaccin est basée sur une comparaison entre les résultats des mesures obtenus pour le vaccin et les résultats des mesures antérieure correspondante du même type de vaccin ou d'autre type de vaccin, le même type de mesure d'activité immunologique étant utilisé pour obtenir les résultats des mesures pour le vaccin et les résultats des mesures antérieures.

**47.** Procédé selon l'une quelconque des revendications précédentes, dans lequel l'antigène de protéine moléculaire est un allergène.

**48.** Procédé selon la revendication 47, dans lequel l'allergène est choisi parmi le groupe constitué d'allergènes de pollen d'arbres, d'allergènes de pollen de graminées, d'allergènes de pollen d'herbes, d'allergènes d'acariens, d'allergènes de venin, d'allergènes de poils d'animaux et d'allergènes de pellicules et d'allergènes alimentaires.

**49.** Procédé selon la revendication 48, dans lequel l'allergène est un allergène de pollen de graminée.

**50.** Procédé selon la revendication 48, dans lequel l'allergène est un allergène de poussières et d'acariens.

**51.** Procédé selon l'une quelconque des revendications 47-50, dans lequel l'allergène est un mélange d'au moins deux espèces d'allergènes différentes.

**52.** Procédé selon l'une quelconque des revendications 47-51, dans lequel l'allergène est sous la forme d'un extrait, d'un allergène purifié, d'un allergène modifié ou d'un allergène recombiné ou d'un mutant d'un allergène recombiné ou de toute combinaison de ceux-ci.

**53.** Procédé selon l'une quelconque des revendications 47-52, dans lequel l'allergène est sous la forme d'un extrait.

**54.** Procédé selon la revendication 53, dans lequel l'activité immunologique de deux ou plus de deux allergènes majeurs et/ou mineurs de l'extrait sont mesurés.

**55.** Procédé selon la revendication 54, dans lequel on mesure en plus l'activité immunologique de l'extrait entier.

**56.** Procédé de préparation d'une préparation de vaccin sous la forme d'un mélange d'antigène de protéine moléculaire et d'un support en phase solide, sous la forme d'hydroxyde d'aluminium, dans lequel le mélange comprend une phase liquide et une phase solide, auquel au moins une partie de l'antigène est attachée, le procédé comprenant

i) le mélange de l'antigène et du support,
ii) la mesure de l'activité immunologique du vaccin en utilisant le procédé selon l'une quelconque des revendications 1-54, et
iii) facultativement, la répétition des étapes i) et ii) jusqu'à ce qu'une activité immunologique souhaitée soit obtenue.

# Inhibition of the binding of IgE to biotinylated Phl p by Phl p or Alutard formulations.

FIG. 1

FIG. 2

FIG. 3

Early T-cell activation by Phl p vaccines
6 hours

Fig. 4

Alutard desorption.
Phl p 1

FIG. 5

Alutard desorption.
Phl p 5

FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- SU 1746318 A
- US 4127385 A, Weeke
- WO 9947680 A
- WO 0240676 A

**Non-patent literature cited in the description**

- **KILDSGAARD et al.** Assessment of the in vivo allergenic potency of new allergy vaccines by intradermal testing in sensitised mice, Clinical Immunology and Allergy in Medicine. *Proceedings of the 21st EAACI Congress,* 2002
- **CHANG et al.** *Vaccine,* 2001, vol. 19, 2884-2889
- **SHI et al.** *Vaccine,* 2002, vol. 20, 80-85
- **LYER et al.** *Vaccine,* 2003, vol. 21, 1219-1223
- **KATZ et al.** *Journal of Virological Methods,* 1989, vol. 25, 101-108
- **THRAENHART et al.** *Journal of Biological Standardisation,* 1989, vol. 17, 291-309